# EUROPEAN PATENT SPECIFICATION

(11) **EP 4 216 230 B1**
(45) Date of publication and mention of the grant of the patent: **06.05.2026**
(21) Application number: 21869805.8
(22) Date of filing: 17.09.2021
(51) Int. Cl.: G06T 7/33, G16H 30/40, G16H 30/20, G16H 10/00, G16H 80/00, A61C 9/00, G06T 3/14, G16H 50/50, G06T 17/00, G06T 17/20, G06T 19/20

(54) **THREE-DIMENSIONAL SCAN DATA PROCESSING SYSTEM AND THREE-DIMENSIONAL SCAN DATA PROCESSING METHOD**
DATENVERARBEITUNGSSYSTEM MIT DREIDIMENSIONALER ABTASTUNG UND DATENVERARBEITUNGSVERFAHREN MIT DREIDIMENSIONALER ABTASTUNG
SYSTÈME DE TRAITEMENT DE DONNÉES DE BALAYAGE TRIDIMENSIONNEL ET PROCÉDÉ DE TRAITEMENT DE DONNÉES DE BALAYAGE TRIDIMENSIONNEL

(30) Priority: 21.09.2020 KR 20200121811; 23.07.2021 KR 20210096777
(43) Date of publication of application: 26.07.2023
(73) Proprietor: Medit Corp., Seoul 07207 (KR)
(72) Inventor: KANG, Dong Hwa, Seoul 02842 (KR); SONG, Myoung Woo, Seoul 02842 (KR)
(74) Representative: Maiwald GmbH
(86) International application number: PCT/KR2021/012870
(87) International publication number: WO 2022/060183

(56) References cited:
- KR-A- 20180 095 972
- KR-B1- 102 121 992
- US-A1- 2009 316 966
- US-A1- 2020 037 848
- US-A1- 2020 202 528
- US-A1- 2020 205 943
- TASAKA AKINORI ET AL: "Applying intraoral scanner to residual ridge in edentulous regions: in vitro evaluation of inter-operator validity to confirm trueness", vol. 19, no. 1, 1 December 2019 (2019-12-01), GB, XP093203765, ISSN: 1472-6831, Retrieved from the Internet <URL:http://link.springer.com/article/10.1186/s12903-019-0918-y/fulltext.html> DOI: 10.1186/s12903-019-0918-y
- STADERINI EDOARDO ET AL: "Three-dimensional prediction of roots position through cone-beam computed tomography scans-digital model superimposition: A novel method", vol. 22, no. 1, 16 December 2018 (2018-12-16), DK, pages 16 - 23, XP093203766, ISSN: 1601-6335, Retrieved from the Internet <URL:https://onlinelibrary.wiley.com/doi/full-xml/10.1111/ocr.12252> DOI: 10.1111/ocr.12252
- TIM JODA ET AL: "The virtual patient in dental medicine", CLINICAL ORAL IMPLANTS RESEARCH, MUNKSGAARD INTERNATIONAL PUBLISHERS, DK, vol. 26, no. 6, 26 March 2014 (2014-03-26), pages 725 - 726, XP071770205, ISSN: 0905-7161, DOI: 10.1111/CLR.12379
- JODA TIM ET AL: "Digital technology in fixed implant prosthodontics", vol. 73, no. 1, 21 December 2016 (2016-12-21), DK, pages 178 - 192, XP093203767, ISSN: 0906-6713, Retrieved from the Internet <URL:https://api.wiley.com/onlinelibrary/tdm/v1/articles/10.1111%2Fprd.12164> DOI: 10.1111/prd.12164
- FERRANDO-CASCALES �LVARO ET AL: "A facially driven complete-mouth rehabilitation with ultrathin CAD-CAM composite resin veneers for a patient with severe tooth wear: A minimally invasive approach", THE JOURNAL OF PROSTHETIC DENTISTRY, ELSEVIER, AMSTERDAM, NL, vol. 123, no. 4, 2 August 2019 (2019-08-02), pages 537 - 547, XP086122663, ISSN: 0022-3913, [retrieved on 20190802], DOI: 10.1016/J.PROSDENT.2019.04.011
- LEE SO-HYOUN ET AL: "Fundamental understandings of mesh process and various formats of digital data in digital dentistry", JOURNAL OF DENTAL IMPLANT RESEARCH, vol. 40, no. 1, 30 March 2021 (2021-03-30), pages 13 - 16, XP093200290, ISSN: 1229-7038, DOI: 10.54527/jdir.2021.40.1.13

## Description

### TECHNICAL FIELD

The present disclosure relates to a three-dimensional scan data processing system and a three-dimensional scan data processing method and, more specifically, to a three-dimensional scan data processing system and a three-dimensional scan data processing method for integrating and using three-dimensional shape data of an object structure scanned at different times.

### BACKGROUND

With the development of the performance of three-dimensional scanners, various types of three-dimensional scanners are being developed and used. In addition, the use of three-dimensional scanners is gradually increasing in dental treatment. US 2009/0316966 A1 describes a method and apparatus for combining 3D dental scans with other 3D data sets, specifically matching a first data set including digital, three-dimensional, dental models with a second data set, including digital cranio-facial 3D medical scan records for improving missing or inaccurate portions of the cranio-facial 3D medical scan record.

Various types of three-dimensional shape data are generated according to the type of three-dimensional scanner and the type of application that drives the three-dimensional scanner. Therefore, there is a need for a method capable of effectively integrating three-dimensional shape data of the structure of an object, generated in environments different from one another.

For example, if three-dimensional shape data can be generated by importing three-dimensional data of a mouth structure scanned during previous treatment of a patient at another dental clinic and integrating the imported three-dimensional data with new three-dimensional data using a three-dimensional scanner, it is possible to simplify an intraoral scan procedure and save time. In addition, according to this method, it is possible to improve various treatment procedures or to develop new treatment methods.

Therefore, for various reasons, such as different types of three-dimensional scanners or different types and formats of driving applications, there is a need for a device and method capable of obtaining a three-dimensional shape of an object by importing existing object scan data of different file formats and effectively integrating the data with new scan data.

### SUMMARY

The present disclosure has been devised to satisfy the above-mentioned needs, and an aspect of the present disclosure is to provide a three-dimensional scan data processing system and a three-dimensional scan data processing method capable of effectively integrating and using three-dimensional shape data of an object scanned or acquired at different times. The invention is defined by the claims.

In order to achieve the above aspect, a three-dimensional scan data processing method of the present disclosure may include an import data reception step in which a data processing device imports import data including shape information of at least a part of an object; a scan data reception step in which a scan data reception module generates, by scanning the object with a three-dimensional scanner, scan data that includes shape information and feature information of the object and is at least partially in common with the import data; an extraction step in which an extraction module extracts a region common to the scan data from the import data; an alignment step in which an alignment module performs alignment based on the region common to the import data and the scan data; and an integration step in which an integration module generates integrated data by integrating the import data and the scan data.

In addition, a three-dimensional scan data processing system of the present disclosure may include: a data processing device configured to import import data defining a three-dimensional shape and including shape information of at least a part of an object; a scan data reception module configured to generate, by scanning the object with a three-dimensional scanner, scan data that includes shape information and feature information of the object and is at least partially in common with the import data; an extraction module configured to extract a region common to the scan data from the import data; an alignment module configured to perform alignment based on the region common to the import data and the scan data; and an integration module configured to generate integrated data by integrating the import data and the scan data.

The three-dimensional scan data processing system and the three-dimensional scan data processing method of the present disclosure may have an effect of easily integrating shape data related to a three-dimensional structure of an object that have been obtained at different times.

The three-dimensional scan data processing system and the three-dimensional scan data processing method of the present disclosure make it possible to easily integrate new shape data of an object with previously acquired shape data of a three-dimensional structure of the same object. Accordingly, the present disclosure has the effect of simplifying a three-dimensional scan procedure, saving time, and enabling improvement of various procedures.

### BRIEF DESCRIPTION OF DRAWINGS

FIG. 1 is a block diagram of a three-dimensional scan data processing system according to an embodiment of the present disclosure.
FIG. 2 is a flowchart illustrating an embodiment of a three-dimensional scan data processing method according to the present disclosure.
FIGS. 3 to 8 illustrate a process of implementing a three-dimensional scan data processing method of the present disclosure by using the three-dimensional scan data processing system illustrated in FIG. 1.

### DETAILED DESCRIPTION

Various types of three-dimensional data are generated according to the type of three-dimensional scanner and the type of application that drives the three-dimensional scanner. Applications form different types of three-dimensional data, and the formed three-dimensional data are not compatible between the applications. The reason for mutual incompatibility is that each application has a unique three-dimensional data format but the three-dimensional data format is not disclosed. Accordingly, when a second application wants to read three-dimensional data generated by a first application, the first application needs to convert the three-dimensional data into a format enabling the second application to read the three-dimensional data, and export the three-dimensional data. Commonly used formats of three-dimensional data include formats with extensions such as OBJ, PLY, and STL, but are not limited thereto. Three-dimensional data in a commonly used format can be read by various applications because the format of the data is open. However, three-dimensional data in a commonly used format often contains only geometry information of the three-dimensional data. Therefore, in order to align three-dimensional data in a general format imported by an application with scan data newly generated by the application, a process of processing the import data is required.

Hereinafter, a three-dimensional scan data processing system and a three-dimensional scan data processing method according to embodiments of the present disclosure will be described with reference to the accompanying drawings.

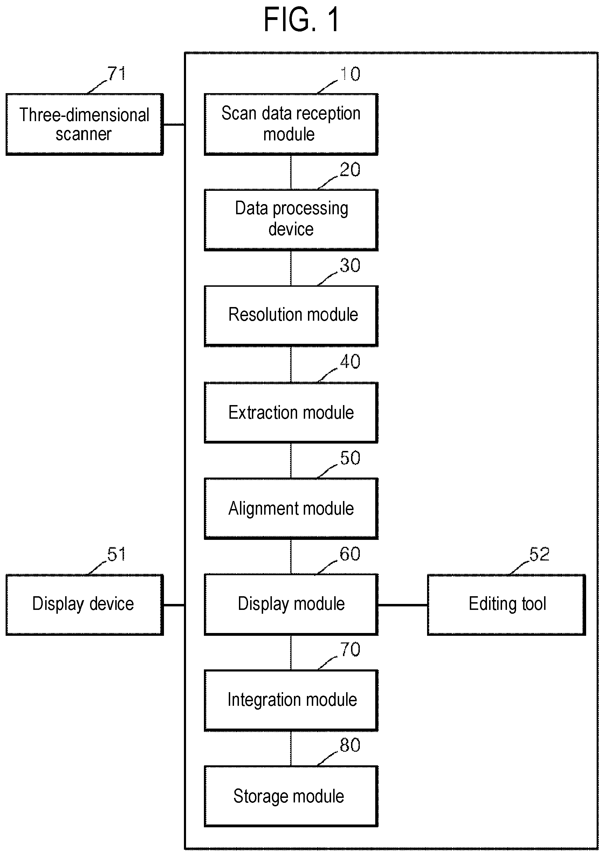

FIG. 1 is a block diagram of a three-dimensional scan data processing system according to an embodiment of the present disclosure.

The present disclosure is aimed at integrating, with a three-dimensional data file of the structure of an object exported by an application, three-dimensional data of the same object newly acquired by scanning. In this case, the application performing the exporting and an application performing importing may be the same application.

In the present disclosure, an "object" is an object to be scanned, and may include a person, an animal, or a part thereof. For example, the object may include a body part (viscera or organ, etc.), an artificial structure attachable on the object or insertable into the object, and the like. For example, the object may include teeth, gingiva, at least a partial region of the mouth, and/or artificial structures (e.g., orthodontic devices including a bracket and a wire, dental restorations including implants, artificial teeth, inlay, onlay, etc., orthodontic aids insertable into the mouth, and the like) insertable into the mouth, teeth or gingiva to which artificial structures are attached, and the like.

In the present disclosure, "data" may refer to information necessary to represent an object in two dimensions or three dimensions. Also, in the present disclosure, "data" may refer to information indicating three-dimensional characteristics of an object including at least one of teeth, gingiva, and an artificial structure attached to the teeth or the gingiva.

Hereinafter, a three-dimensional data file regarding the structure of an object exported by an application is referred to as import data, and newly acquired three-dimensional data of the structure of the object, which is to be integrated with the import data, is referred to as scan data.

The import data may refer to data that is exported by an application and has an open file format. The import data may be data having geometry information of at least one of point data and mesh data. The import data may be, for example, a file having an extension such as OBJ, PLY, or STL, but the file format is not limited thereto.

The scan data may be data acquired from a three-dimensional scanner and partially having a mesh shape. After raw data is acquired from the three-dimensional scanner and transmitted to the application, upon being stored in the form of a complete mesh in which all points are connected, the raw data has a unique data format. Here, the scan data may be data before being stored in the form of a complete mesh. The scan data may be data in which feature information defining the feature of the three-dimensional shape of an object is further included in the raw data.

It is assumed that such feature information is not included in import data, but in some cases, such feature information may also be partially included in the import data.

Hereinafter, an example in which an object to be three-dimensionally scanned is a human mouth will be described.

Import data and scan data are different types of three-dimensional shape data of the mouth of the same person. That is, the import data and the scan data each include a common mouth shape part. The import data may be three-dimensional shape data having various formats. The scan data is three-dimensional shape data having a different file format from import data. The scan data includes point data, mesh data, and feature information, and may be generated in real time.

A three-dimensional scanner 71 used in a three-dimensional scan data processing system according to the present embodiment may include a medical device for acquiring shape data of an object. For example, the three-dimensional scanner 71 may be an intraoral scanner or a table scanner that scans an object by using at least one image sensor (e.g., an optical camera). The three-dimensional scanner 71 may transmit raw data acquired from the object to a scan data reception module 10. When a patient's mouth is scanned by the three-dimensional scanner 71, the scan data reception module 10 processes the raw data acquired from the three-dimensional scanner 71 to generate scan data including feature information and points defining a three-dimensional shape. That is, the scan data is obtained from data acquired from the three-dimensional scanner 71.

The three-dimensional scan data processing system of the present embodiment includes at least one among the scan data reception module 10, a data processing device 20, an alignment module 50, a display module 60, an editing tool 52, and an integration module 70. The scan data reception module 10 may be included in the three-dimensional scanner 71 according to an embodiment. The three-dimensional scan data processing system according to an example may be a computing device such as a smartphone, a laptop computer, a desktop computer, a PDA, and a tablet PC, but is not limited thereto.

Import data is received by the data processing device 20. The data processing device 20 may receive import data in the form of a file. The data processing device 20 may receive scan data in the form of data obtained by processing the raw data obtained from the three-dimensional scanner 71. The import data and the scan data basically define the shape of an object by three-dimensional coordinates of points positioned on the surface of the object. In addition to the coordinates of these points, the import data and the scan data may further include information on meshes formed by connecting adjacent points, and may further include normal vector information indicating the direction of each point.

Also, feature information included in the scan data is information indicating the feature of a three-dimensional shape of an object calculated by using points of the object obtained by using a three-dimensional scanner. A surface curvature formed by connecting adjacent points of the scan data may be an example of feature information of the scan data. A corrugation shape of a specific point on the surface of the object defined by the scan data may be feature information of the scan data.

A storage module 80 stores the import data received by the data processing device 20 and the scan data generated by the scan data reception module 10.

The extraction module 40 extracts a region common to the scan data from the import data. The extraction module 40 generates at least one piece of feature information as described above, such as surface curvature information, surface corrugation information, and user input information, from the import data to extract a region common to the scan data.

The alignment module 50 adjusts the position and direction of at least one of the import data and the scan data, based on the region which the import data and the scan data have in common, and aligns one with the other.

The integration module 70 integrates the import data and the scan data aligned with each other, and the import data and the scan data are stored as integrated data by the storage module 80.

The display module 60 displays a mouth shape 102, defined by the import data received by the data processing device 20 and the scan data generated by the scan data reception module 10, on a display device 51 such as a monitor. Alternatively, the display module 60 displays, on the display device 51, a mouth shape 101 defined by the import data aligned by the alignment module 50 and the mouth shape 102 defined by the scan data. A monitor and a VR device may be examples of the display device 51.

The editing tool 52 edits the import data or the scan data according to a user's command that is input through an input device such as a mouse, a touch pad, a keyboard, and an input module provided in a scanner. In the case of the present embodiment, the editing tool 52 is displayed on the display device 51 together with the shape of the mouth by the display module 60. The editing tool 52 receives a command from an input device and provides editing functions such as deletion of parts of the import data and the scan data.

A resolution module 30 converts the import data into a form suitable for integration with the scan data. A specific operation will be described below.

Hereinafter, a specific operation of the above-described device for implementing a three-dimensional scan data processing method, and the three-dimensional scan data processing method according to the present disclosure will be described in detail.

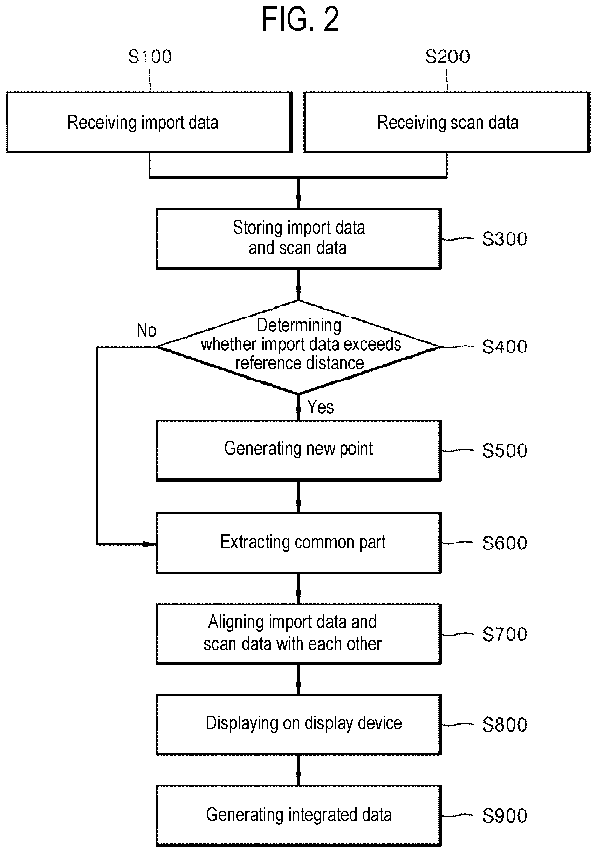

FIG. 2 is a flowchart illustrating an embodiment of a three-dimensional scan data processing method according to the present disclosure.

First, import data is imported by the data processing device 20 (an import data reception step S 100). In the case of the present embodiment, the import data is configured to define the three-dimensional shape of a mouth by using points in a three-dimensional space and polygonal meshes formed by connecting the points. In general, in the field of intraoral scanning, a triangular mesh is mainly used. In present embodiment, a description will be made by using, as an example, import data in the form of triangular meshes formed by connecting adjacent points in the three-dimensional space. In some cases, the import data may not include mesh information, but only point information. In addition, the import data may additionally include normal vector information indicating a direction of each point.

The import data may be previously scanned and stored data, or may be data acquired by scanning performed by another three-dimensional scanner 71 or another device. In present embodiment, an example in which the import data reception step is performed in a manner in which the data processing device imports the import data without using the three-dimensional scanner will be described.

Scan data to be described later includes data defining a three-dimensional shape of the mouth with points in a three-dimensional space and polygonal meshes (triangular meshes in the present embodiment) formed by connecting the points. The scan data is newly scanned data to be added to or accumulated with the import data. The scan data has a different format from the import data, and thus may include a reference direction, a resolution, and the like that are different from those of the import data, and may further include the feature information as described above.

In the present embodiment, an example in which coordinates of points in both import data and scan data are stored in units of millimeters (mm) will be described.

The storage module 80 stores the import data received by the data processing device 20 (S300).

The resolution module 30 determines whether a distance between adjacent points of the import data exceeds a reference distance (a resolution determination step S400). The resolution determination step may be performed with respect to all points of the import data or only with respect to some sampled points.

The reference distance indicates the maximum allowable distance between adjacent points of the scan data. Alternatively, the reference distance may indicate a distance between adjacent voxels when the scan data has been voxelized. The reference distance becomes a criterion for determining the resolution of a mouth shape in the scan data. As the distance between adjacent points of the scan data becomes narrower, the shape of the mouth is defined with higher resolution. In the case of the present embodiment, the reference distance is 0.1 mm. That is, the points of the scan data are configured so that the distance between adjacent points is within 0.1 mm, and the size of a mesh is determined accordingly.

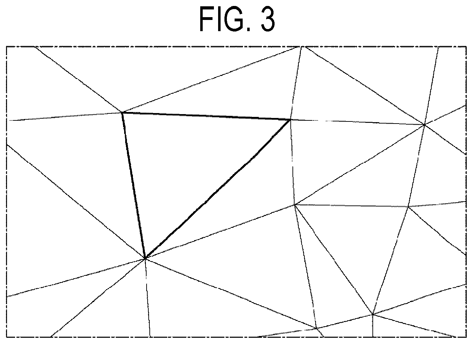

FIG. 3 illustrates a part of a mesh structure of import data. In the present embodiment, points of the import data are distributed at intervals of 0.4 mm. Since the interval between points of the scan data is 0.1 mm, the reference distance is 0.1 mm in the present embodiment. The resolution module 30 performs the resolution determination step to determine that the distance between adjacent points of the import data exceeds the reference distance.

In the above-described resolution determination step, when it is determined that the distance between adjacent points of the import data exceeds the reference distance, the resolution module 30 updates the import data by generating new points by dividing at least some meshes so that the distance between adjacent points of the import data is equal to or less than the reference distance (an import data updating step S500). As such, the resolution module 30 formally adjusts the resolution of the import data in order to facilitate alignment and integration of the import data with the scan data.

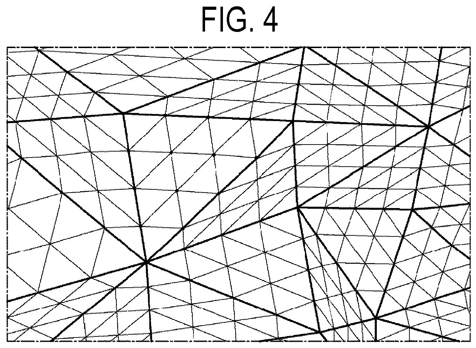

FIG. 4 illustrates the mesh structure of the import data after completion of the import data updating step. The resolution module 30 generates a new mesh by adding new points at 0.1mm intervals between the existing points of the import data and dividing the mesh so that the points are connected. Through this process, the import data is converted into data in which at least some points are arranged at intervals within the reference distance, as in the scan data. The resolution module 30 converts the import data in this way and updates the converted import data to new import data. The storage module 80 stores the updated import data.

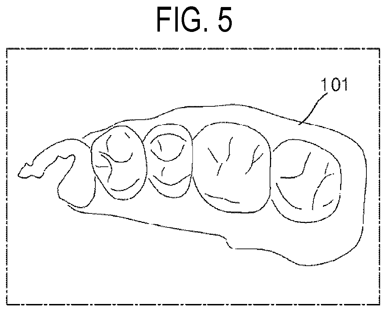

As described above, when the resolution module 30 completes the determination of resolution of the import data and the generation of new points, the display module 60 displays, on the display device 51, the three-dimensional shape 101 of the mouth defined by the import data (a display step). The scan data processing device according to the present embodiment may voxelize the three-dimensional shape 101 of the mouth defined by the import data and may display the voxelized shape on the display device 51. FIG. 5 illustrates a state in which the shape 101 based on the import data is displayed on the display device 51.

In the previous resolution determination step S400, when it is determined that the distance between adjacent points of the import data does not exceed the reference distance, a scan data reception step and subsequent processes are performed without performing the import data updating step (S500).

Next, the scan data reception module 10 performs a process of generating scan data (a scan data reception step S200). The scan data reception module 10 may receive scan data by importing a scan data file, but in the present embodiment, an example of generating scan data which is a type of data accumulated in real time through the three-dimensional scanner 71 will be described. When a patient's mouth is scanned by the three-dimensional scanner 71, the scan data reception module 10 processes raw data acquired by the three-dimensional scanner 71 to generate scan data including feature information and points that define a three-dimensional shape. The scan data reception module 10 calculates and generates the feature information of the scan data as described above. As described above, the scan data may further include normal vector information and/or mesh information configured by connecting points. The scan data reception module 10 receives the raw data generated by the three-dimensional scanner 71 in real time. The storage module 80 stores the scan data generated by the scan data reception module 10 (S300).

The extraction module 40 calculates feature information of the import data by using the coordinates of points of the import data, and extracts common parts of the import data and the feature information of the scan data (an extraction step S600). The alignment module 50 aligns the import data and the scan data by making common regions of the import data and scan data correspond to each other (an alignment step S700). That is, the alignment module 50 performs an alignment operation by changing the position and direction of one of the import data and the scan data to match the other.

The alignment module 50 aligns the import data and the scan data by using common mouth shape parts of the scan data and the import data extracted by the extraction module 40.

The import data and the scan data are arranged in different positions and directions in a three-dimensional space when reference coordinates are different at the time of generation of the import data and the scan data. This phenomenon occurs when devices, environments, and programs for generating import data and scan data are different.

In order to accumulate or integrate and use import data and scan data, import data and scan data generated based on different coordinates should be aligned with each other and converted into a data format based on the same coordinates. The present disclosure uses common mouth shape parts of import data and scan data to align the import data and the scan data with each other. In this way, various methods may be used to align import data and scan data with each other using a common shape of the import data and the scan data.

In some cases, the extraction module 40 may extract common parts of import data and scan data by mapping import data and scan data to each other by using, as feature information, a common point of tooth surface corrugation in the mouth shape 101 defined by the import data and the mouth shape 102 defined by the scan data. The extraction module 40 may automatically identify a fine corrugation pattern or a characteristically protruding or recessed part on the surface of a tooth according to the structure of the tooth, and may map the import data and the scan data based on common points thereof.

Also, in a similar way, a surface curvature formed by connecting points located adjacent to each other may be stored or calculated as feature information of the three-dimensional shape of the object, and the extraction module 30 may extract common parts of import data and scan data by identifying common points of the import data and feature information of the scan data and mapping the common points to each other.

For example, the extraction module 40 and the alignment module 50 may find corresponding planes of import data and scan data and align the corresponding planes with each other by an Iterative Closest Points (ICP) technique. The extraction module 40 and the alignment module 50 may align and place the import data and the scan data in a three-dimensional space by accurately and precisely matching the import data and the scan data to each other by using the ICP technique of repeatedly finding closest points and matching the closest points to each other.

In addition, the extraction module 40 and the alignment module 50 may use both an alignment method using feature information of the three-dimensional shapes 101 and 102 defined by the scan data and the import data and an alignment method using an ICP technique. For example, the extraction module 40 and the alignment module 50 may primarily perform alignment using feature information and then secondarily perform alignment using the ICP technique.

The extraction module 40 and the alignment module 50 may identify feature information of an object through various other methods in addition to the above-described method, and may perform the extraction step S600 and the alignment step S700, respectively.

As described above, in the case of acquiring scan data in real time by using the three-dimensional scanner 71, when scan data regarding a part common to import data is not obtained, the extraction module 40 and the alignment module 50 stand by without performing extraction and alignment operations. When feature information common to import data starts to be received to accumulated scan data, the extraction module 40 and the alignment module 50 perform extraction and alignment operations.

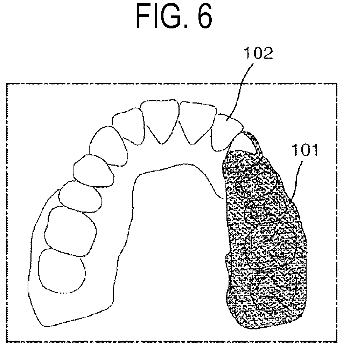

As described above, when the alignment of the import data and the scan data by the alignment module 50 is completed, the display module 60 displays, on the display device 51, the mouth shape 101 based on the import data and the mouth shape 102 based on the scan data (a display step S800). FIG. 6 illustrates a state in which both the mouth shape 101 based on the import data and the mouth shape 102 based on the scan data are displayed on the display device 51. The alignment module 50 aligns the three-dimensional shape 102 of the mouth based on scan data with the three-dimensional shape 101 of the mouth based on import data as illustrated in FIG. 5, and as shown in FIG. 6, the three-dimensional shapes 101 and 102 are displayed on the display device 51 by the display module 60 while overlapping each other.

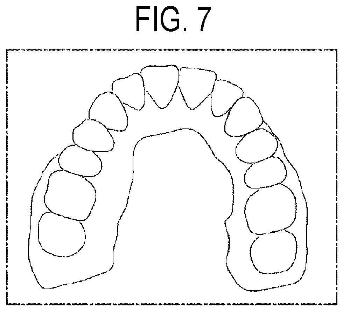

The display module 60 may selectively display the mouth shape 101 based on the import data and the mouth shape 102 based on the scan data on the display device 51 according to a command received through an input device. That is, the display module 60 may display both the import data and the scan data on the display device 51, or may display one thereof or make one thereof invisible. In addition, as illustrated in FIG. 6, the display module 60 may display the shape 101 based on the import data and the shape 102 based on the scan data on the display device 51 so as to be distinguished from each other by having different colors. In addition, as illustrated in FIG. 7, the display module 60 may overlap and display the import data and the scan data on the display device 51 so as not to be distinguished from each other, and may display, on the display device 51, integrated data in which the import data and the scan data are integrated.

In some cases, the display module 60 may voxelize each of the import data and the scan data and display converted data on the display device 51. Various well-known methods may be used as the method in which the display module 60 voxelizes the import data and the scan data and displays the voxelized data on the display device 51. For example, voxelization may be performed by displaying spherical voxels, which have individual points as centers, in a three-dimensional space. At this time, the radius of a sphere corresponding to each point may be changed to various values according to settings.

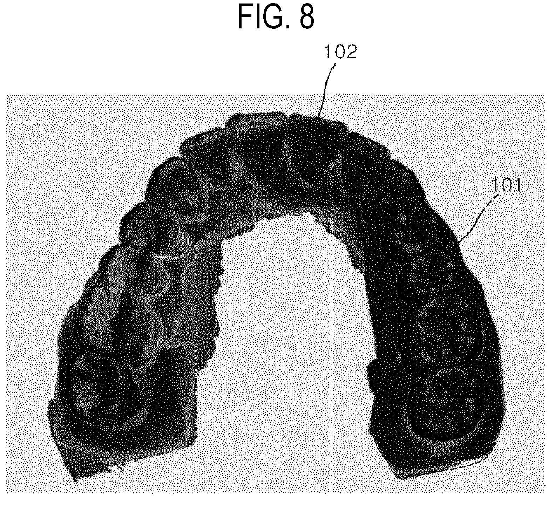

In addition, as illustrated in FIG. 8, the display module 60 may display the integrated data on the display device with a different color for each region according to the reliability of mouth shape data. FIG. 8 illustrates that the display module 60 displays, on a display device, a mouth shape with a first color (green) at a high-reliability part and with a second color (red) at a low-reliability part. Reliability is calculated using factors such as the number of scan shots acquired by the three-dimensional scanner 71 for each region, the scan angle of scan shots, and the density of points acquired for each region. Even when new scan data is input to a part determined to have high reliability, new data may not be added to the three-dimensional shape 102 defined by the scan data. The determination that the reliability is high may indicate that sufficient data has been input to define the corresponding part. Therefore, even when data is continuously acquired by a three-dimensional scanner, new data may not be added to the three-dimensional shape. In the case of scan data, reliability may be calculated using factor information, but in the case of import data, accurate reliability calculation is impossible because factor information is insufficient. Accordingly, predetermined reliability may be given to the three-dimensional shape 101 defined by the import data. In some cases, the three-dimensional shape 101 defined by the import data may be given high reliability. When the reliability is given as a numerical value, the reliability of the three-dimensional shape 101 defined by the import data may be set to 100, which is the highest value. When the reliability of the three-dimensional shape 101 defined by the import data is set to 100, the three-dimensional shape 101 may be maintained without being filtered (deleted) in the case of applying a function for filtering a region having low reliability.

In addition, the display module 60 may perform the display step S800 such that scan data generated and accumulated in real time is displayed on the display device 51 while being updated in real time. In this way, when the scan data reception module 10 generates scan data in real time, the alignment module 50 and the display module 60 perform the alignment step S700 and the display step S800, respectively, while updating accumulated scan data in real time.

When the steps of importing the scan data or accumulating, aligning, and displaying the scan data by the three-dimensional scanner 71 are completed, the integration module 70 integrates the import data and the scan data converted by the alignment module 50, and the import data and the scan data may be stored as integrated data by the storage module 80 (an integration step S900). The integration module 70 may generate integrated data by simply combining the import data and the scan data, or may generate new integrated data by arranging points and meshes by performing numerical calculations on the import data and the scan data. That is, the integration module 70 may generate new integrated data by converting the import data and the scan data by merging closest points in a mouth shape part where the import data and scan data overlap each other or generating a new point at an appropriate location.

In the resolution determination step S400 and the import data updating step S500 described above, the processes of comparing the distance between adjacent points of the import data with the reference distance and adjusting the resolution of the import data have been performed. Therefore, in the integration step S900, it is possible for the integration module 70 to effectively generate integrated data without being subtantially affected by the overall resolution.

Three-dimensional data of the shape of an object structure may be easily obtained by effectively integrating different types of three-dimensional shape data of the structure of the same object by using the above-described method. According to the present disclosure, it is possible to effectively integrate three-dimensional data related to an object structure and obtained by systems having different formats and conditions. In addition, it is possible to generate integrated data by easily aligning shape data of an object partially obtained at different times.

According to the three-dimensional scan data processing method according to the present invention, a plurality of two-dimensional images obtained in the process of using the three-dimensional scanner 71 may not be directly used but may be integrated and stored in the form of integrated data including points and feature information as described above, thereby enabling effective use for future treatment such as correction, implantation, and tooth extraction while reducing the volume of data on the shape of the mouth.

Although the present disclosure has been described with an exemplary example, the scope of the present disclosure is not limited to the form described and illustrated above.

For example, it is also possible to edit, using the editing tool 52, the import data received by the data processing device 20 in the import data reception step S100 and then perform the scan data reception step and subsequent processes to generate integrated data. In this case, after the import data receiving step S100 is performed, the editing tool 52 receives an editing command for editing the import data and updates the import data (an editing step). The editing step is performed through the editing tool 52. The editing tool 52 is displayed on the display device 51 together with a mouth shape of the import data, and a user selects and deletes a part of the import data, which is unnecessary or needs to be updated, by using an input device such as a mouse. The storage module 80 stores the import data updated in the editing step, and the integration step S900 is performed after the editing step is completed. After a part of the import data has been deleted in this way, it is possible to easily integrate the import data with the scan data. This method may be effectively used when a part of the import data is determined to be inaccurate. For example, in the case of import data obtained by scanning of a patient's mouth immediately after surgical treatment of the patient's mouth, the gums at the surgical site may is swollen. In this case, more accurate mouth shape data may be obtained easily and quickly by deleting the swollen gum part of the import data by means of the editing tool 52 and scanning the scan data by scanning again the gum where the swelling has subsided.

In addition, it has been described that in the alignment step S700, the alignment module aligns import data and scan data by using surface curvature or common corrugation as feature information. However, various other methods in addition to this method may be used, or feature information may be calculated by using two or more methods in combination, and the alignment step S700 may be performed by an alignment module.

In some cases, it is also possible to use a method in which the data processing device 20 receives, as feature information, places identical to each other in the mouth shapes 101 and 102 of the import data and the scan data from the user through an input device. That is, first, the data processing device 20 receives each of at least one common point at places identical to each other in the three-dimensional shapes 101 and 102 of the mouth, respectively defined by the import data and the scan data displayed on the display device 51, as feature information through an input device. An example of the above-described method may be a method for clicking each of common points of the mouth shapes 101 and 102 based on the import data and the scan data by using an input device such as a mouse. Next, the alignment module 50 performs the alignment step S700 by aligning the import data and the scan data by mapping, as feature information, the common points of the import data and the scan data received by the data processing device 20. In addition, in the aligning step S700, the import data and the scan data are primarily aligned using the common points of the import data and the scan data as feature information, and then secondarily aligned using the ICP technique.

In addition, the order of performing the scan data reception step to the import data updating step described above is not limited to the order described above, but can be variously modified according to the user's selection or the configuration of hardware and software configured to perform the present disclosure. For example, the import data received by the data processing device 20 in the import data reception step may be first displayed on the display device 51 in a display step, may be divided with new points generated through the resolution determination step and the import data updating step, and then may be displayed again on the display device 51 in the display step.

When a common part that can be aligned with the import data is found in the alignment step while the scan data generated by the scan data reception module 10 in the scan data reception step is displayed on the display device 51 in real time in the display step, the display step may be performed by simultaneously displaying the import data and the scan data.

In addition, the process of comparing the distance between adjacent points of the import data with the reference distance by performing the resolution determination step has been described above. However, in some cases, the three-dimensional scan data processing method of the present disclosure may be performed by omitting the resolution determination step and the import data updating step. In this case, the three-dimensional scan data processing system that performs the three-dimensional scan data processing method may be configured without a resolution module.

In addition, the three-dimensional scan data processing system may be configured to be structured not to include at least one among an alignment module, a display module, an editing tool, and a scan processor.

## Claims

1. A three-dimensional scan data processing method comprising:
an import data reception step (S100) in which a data processing device (20) imports import data comprising shape information of at least a part of an object;
a scan data reception step (S200) in which a scan data reception module (10) generates, by scanning the object with a three-dimensional scanner (71), scan data that comprises shape information and feature information of the object and is at least partially in common with the import data;
an extraction step (S600) in which an extraction module (40) extracts, from the import data, a region common to the scan data;
an alignment step (S700) in which an alignment module (50) performs alignment based on the region common to the import data and the scan data; and
an integration step (S900) in which an integration module (70) generates integrated data by integrating the import data and the scan data,
wherein in the extraction step (S600), the extraction module (40) generates at least one selected from a group of surface curvature information and surface corrugation information formed by connecting adjacent points of the import data, as feature information of a three-dimensional shape of the object, and
wherein in the alignment step (S700), the import data and the scan data are aligned with each other by identifying common points based on the import data and the feature information of the scan data and mapping the common points to each other.

2. The three-dimensional scan data processing method of Claim 1, wherein the extraction step (S600) comprises generating, by the extraction module (40), the feature information of the three-dimensional shape of the object from the import data, and
the feature information comprises at least one selected from a group of the surface curvature information, the surface corrugation information, and user input information.

3. The three-dimensional scan data processing method of Claim 1, further comprising a display step (S800) in which a display module (60) displays, on a display device (51), the three-dimensional shape of the object defined by at least one selected from a group of the import data and the scan data.

4. The three-dimensional scan data processing method of Claim 3, further comprising an editing step in which an editing tool (52) receives an editing command to edit a part of the import data and updates the import data,
wherein the integration step (S900) is performed after the editing step is performed.

5. The three-dimensional scan data processing method of Claim 3, wherein in the display step (S800), the display module (60) selectively displays the three-dimensional shapes of the object, which are defined by the import data and the scan data, on the display device (51) according to a command received through an input device.

6. The three-dimensional scan data processing method of Claim 3, wherein in the display step (S800), the display module (60) voxelizes each of the import data and scan data and displays the voxelized data on the display device (51).

7. The three-dimensional scan data processing method of Claim 3, wherein in the display step (S800), the three-dimensional shapes of the object, which are defined by the import data and the scan data, are displayed on the display device (51) in different colors.

8. The three-dimensional scan data processing method of Claim 3, wherein in the display step, the display module (60) displays each region on the display device (51) in a different color according to reliability of data, and the display module (60) displays a part corresponding to the import data on the display device (51) according to predetermined reliability, and
reliability of the import data is set to a highest value in a range of predetermined numerical values.

9. The three-dimensional scan data processing method of Claim 1, wherein in the import data reception step (S100), the data processing device (20) further receives, as the import data, at least one selected from a group of a normal vector indicating directions of points of the import data and meshes formed by connecting the points.

10. The three-dimensional scan data processing method of Claim 1, further comprising:
a resolution determination step (S400) in which a resolution module (30) determines whether a distance between adjacent points of the import data exceeds a reference distance, which is a maximum allowable distance between adjacent points of the scan data; and
an import data updating step (S500) in which, when the distance between the adjacent points of the import data is determined to exceed the reference distance in the resolution determination step (S400), the resolution module (30) updates the import data by generating new points between at least some points such that the distance between the adjacent points of the import data is equal to or less than the reference distance,
wherein the integration step (S900) is performed after the resolution determination step (S400) and the import data updating step (S500) are performed.

11. The three-dimensional scan data processing method of Claim 1, further comprising a common point reception step in which an input module receives, through an input device, at least one common point at places identical to each other in the three-dimensional shapes of the object defined by the import data and the scan data,
wherein in the alignment step (S700), the alignment module (50) aligns the import data and the scan data with each other by a method in which the input module maps the common point of the import data and the scan data that is received in the common point reception step.

12. The three-dimensional scan data processing method of Claim 1, wherein the scan data generated by the scan data reception module (10) in the scan data reception step (S200) is data accumulated in real time by the three-dimensional scanner (71),
the alignment step (S700) and the integration step (S900) are performed while the scan data accumulated in real time by the scan data reception step (S200) is updated, and
in the import data reception step (S100), the data processing device (20) receives the import data without using the three-dimensional scanner (71),

13. A three-dimensional scan data processing system comprising:
a data processing device (20) configured to import import data defining a three-dimensional shape including shape information of at least a part of an object;
a scan data reception module (10) configured to generate, by scanning the object with a three-dimensional scanner, scan data that comprises shape information and feature information of the object and is at least partially in common with the import data;
an extraction module (40) configured to extract a region common to the scan data from the import data;
an alignment module (50) configured to perform alignment based on the region common to the import data and the scan data; and
an integration module (70) configured to generate integrated data by integrating the import data and the scan data,
wherein the extraction module (40) is configured to generate at least one selected from a group of surface curvature information and surface corrugation information formed by connecting adjacent points of the import data, as feature information of a three-dimensional shape of the object, and
wherein the alignment module (50) is configured to align the import data and the scan data by identifying common points based on the import data and the feature information of the scan data and mapping the common points to each other.

14. The three-dimensional scan data processing system of Claim 13, wherein the extraction module (40) generates the feature information of the three-dimensional shape of the object from the import data, and
the feature information comprises at least one selected from a group of the surface curvature information, the surface corrugation information, and user input information.

## Patentansprüche

1. Verfahren zur Verarbeitung dreidimensionaler Scandaten, umfassend:
einen Schritt (S100) zum Empfangen von Importdaten, bei dem eine Datenverarbeitungsvorrichtung (20) Importdaten empfängt, die Forminformationen zumindest eines Teils eines Objekts umfassen;
einen Schritt (S200) zum Empfangen von Scandaten, bei dem ein ScandatenEmpfangsmodul (10) durch Abtasten des Objekts mit einem dreidimensionalen Scanner (71) Scandaten erzeugt, die Forminformationen und Merkmalsinformationen des Objekts umfassen und zumindest teilweise mit den Importdaten übereinstimmen;
einen Extraktionsschritt (S600), in dem ein Extraktionsmodul (40) aus den Importdaten einen Bereich extrahiert, der mit den Scandaten übereinstimmt;
einen Ausrichtungsschritt (S700), in dem ein Ausrichtungsmodul (50) eine Ausrichtung auf der Grundlage des Bereichs durchführt, der den Importdaten und den Scandaten gemeinsam ist; und
einen Integrationsschritt (S900), in dem ein Integrationsmodul (70) integrierte Daten erzeugt, indem es die Importdaten und die Scandaten integriert,
wobei im Extraktionsschritt (S600) das Extraktionsmodul (40) mindestens eine aus einer Gruppe von Oberflächenkrümmungsinformationen und Oberflächenwelleninformationen, die durch Verbinden benachbarter Punkte der Importdaten gebildet werden, als Merkmalsinformationen einer dreidimensionalen Form des Objekts erzeugt, und
wobei im Ausrichtungsschritt (S700) die Importdaten und die Scandaten aufeinander ausgerichtet werden, indem gemeinsame Punkte auf der Grundlage der Importdaten und der Merkmalsinformationen der Scandaten identifiziert und die gemeinsamen Punkte einander zugeordnet werden.

2. Verfahren zur Verarbeitung dreidimensionaler Scandaten nach Anspruch 1, wobei der Extraktionsschritt (S600) umfasst, dass das Extraktionsmodul (40) die Merkmalsinformationen der dreidimensionalen Form des Objekts aus den Importdaten erzeugt, und
die Merkmalsinformationen mindestens eine Information umfassen, die aus einer Gruppe ausgewählt ist, die aus den Oberflächenkrümmungsinformationen, den Oberflächenwelleninformationen und den Benutzereingabeinformationen besteht.

3. Verfahren zur Verarbeitung dreidimensionaler Scandaten nach Anspruch 1, das ferner einen Anzeigeschritt (S800) umfasst, in dem ein Anzeigemodul (60) auf einer Anzeigevorrichtung (51) die dreidimensionale Form des Objekts anzeigt, die durch mindestens eine aus einer Gruppe von Importdaten und Scandaten ausgewählte Information definiert ist.

4. Verfahren zur Verarbeitung dreidimensionaler Scandaten nach Anspruch 3, das ferner einen Bearbeitungsschritt umfasst, in dem ein Bearbeitungswerkzeug (52) einen Bearbeitungsbefehl zum Bearbeiten eines Teils der Importdaten empfängt und die Importdaten aktualisiert,
wobei der Integrationsschritt (S900) nach Durchführung des Bearbeitungsschritts durchgeführt wird.

5. Verfahren zur Verarbeitung dreidimensionaler Scandaten nach Anspruch 3, wobei im Anzeigeschritt (S800) das Anzeigemodul (60) die durch die Importdaten und die Scandaten definierten dreidimensionalen Formen des Objekts auf der Anzeigevorrichtung (51) entsprechend einem über eine Eingabevorrichtung empfangenen Befehl selektiv anzeigt.

6. Verfahren zur Verarbeitung dreidimensionaler Scandaten nach Anspruch 3, wobei im Anzeigeschritt (S800) das Anzeigemodul (60) sowohl die Importdaten als auch die Scandaten voxelt und die voxelten Daten auf der Anzeigevorrichtung (51) anzeigt.

7. Verfahren zur Verarbeitung dreidimensionaler Scandaten nach Anspruch 3, wobei im Anzeigeschritt (S800) die durch die Importdaten und die Scandaten definierten dreidimensionalen Formen des Objekts auf dem Anzeigegerät (51) in unterschiedlichen Farben angezeigt werden.

8. Verfahren zur Verarbeitung dreidimensionaler Scandaten nach Anspruch 3, wobei im Anzeigeschritt das Anzeigemodul (60) jeden Bereich auf der Anzeigevorrichtung (51) entsprechend der Zuverlässigkeit der Daten in einer anderen Farbe anzeigt, und das Anzeigemodul (60) einen den Importdaten entsprechenden Teil auf der Anzeigevorrichtung (51) entsprechend einer vorbestimmten Zuverlässigkeit anzeigt, und
die Zuverlässigkeit der Importdaten auf einen höchsten Wert in einem Bereich vorbestimmter Zahlenwerte gesetzt wird.

9. Verfahren zur Verarbeitung dreidimensionaler Scandaten nach Anspruch 1, wobei im Schritt (S100) zum Empfangen von Importdaten die Datenverarbeitungsvorrichtung (20) ferner als Importdaten mindestens einen aus einer Gruppe von Normalenvektoren, die Richtungen von Punkten der Importdaten angeben, und durch Verbinden der Punkte gebildeten Netzen empfängt.

10. Verfahren zur Verarbeitung dreidimensionaler Scandaten nach Anspruch 1, das ferner umfasst:
einen Auflösungsbestimmungsschritt (S400), in dem ein Auflösungsmodul (30) bestimmt, ob ein Abstand zwischen benachbarten Punkten der Importdaten einen Referenzabstand überschreitet, der ein maximal zulässiger Abstand zwischen benachbarten Punkten der Scandaten ist; und
einen Schritt zur Aktualisierung der Importdaten (S500), bei dem das Auflösungsmodul (30), wenn im Schritt zur Bestimmung der Auflösung (S400) festgestellt wird, dass der Abstand zwischen benachbarten Punkten der Importdaten den Referenzabstand überschreitet, die Importdaten aktualisiert, indem es neue Punkte zwischen zumindest einigen Punkten generiert, sodass der Abstand zwischen benachbarten Punkten der Importdaten gleich oder kleiner als der Referenzabstand ist,
wobei der Integrationsschritt (S900) durchgeführt wird, nachdem der Auflösungsbestimmungsschritt (S400) und der Aktualisierungsschritt für die Importdaten (S500) durchgeführt wurden.

11. Verfahren zur Verarbeitung dreidimensionaler Scandaten nach Anspruch 1, das ferner einen Schritt zum Empfangen gemeinsamer Punkte umfasst, bei dem ein Eingabemodul über eine Eingabevorrichtung mindestens einen gemeinsamen Punkt an Stellen empfängt, die in den durch die Importdaten und die Scandaten definierten dreidimensionalen Formen des Objekts identisch sind,
wobei im Ausrichtungsschritt (S700) das Ausrichtungsmodul (50) die Importdaten und die Scandaten miteinander ausrichtet, und zwar durch ein Verfahren, bei dem das Eingabemodul den im Schritt zum Empfangen gemeinsamer Punkte empfangenen gemeinsamen Punkt der Importdaten und der Scandaten abbildet.

12. Verfahren zur Verarbeitung dreidimensionaler Scandaten nach Anspruch 1, wobei die vom Scandatenempfangsmodul (10) im Scandatenempfangsschritt (S200) erzeugten Scandaten Daten sind, die vom dreidimensionalen Scanner (71) in Echtzeit gesammelt werden,
der Ausrichtungsschritt (S700) und der Integrationsschritt (S900) durchgeführt werden, während die im Scan-Datenempfangsschritt (S200) in Echtzeit gesammelten Scan-Daten aktualisiert werden, und
im Importdatenempfangsschritt (S100) die Datenverarbeitungsvorrichtung (20) die Importdaten ohne Verwendung des dreidimensionalen Scanners (71) empfängt,

13. Dreidimensionales Scandatenverarbeitungssystem, umfassend:
eine Datenverarbeitungsvorrichtung (20), die so konfiguriert ist, dass sie Importdaten importiert, die eine dreidimensionale Form definieren, einschließlich Forminformationen von mindestens einem Teil eines Objekts;
ein Scandatenempfangsmodul (10), das so konfiguriert ist, dass es durch Scannen des Objekts mit einem dreidimensionalen Scanner Scandaten erzeugt, die Forminformationen und Merkmalsinformationen des Objekts umfassen und zumindest teilweise mit den Importdaten übereinstimmen;
ein Extraktionsmodul (40), das so konfiguriert ist, dass es einen Bereich, der den Scandaten gemeinsam ist, aus den Importdaten extrahiert;
ein Ausrichtungsmodul (50), das so konfiguriert ist, dass es eine Ausrichtung auf der Grundlage des gemeinsamen Bereichs der Importdaten und der Scandaten durchführt; und
ein Integrationsmodul (70), das so konfiguriert ist, dass es durch Integration der Importdaten und der Scandaten integrierte Daten erzeugt,
wobei das Extraktionsmodul (40) so konfiguriert ist, dass es mindestens eine der folgenden Informationen aus einer Gruppe von Oberflächenkrümmungsinformationen und Oberflächenwelleninformationen, die durch Verbinden benachbarter Punkte der Importdaten gebildet werden, als Merkmalsinformationen einer dreidimensionalen Form des Objekts erzeugt, und
wobei das Ausrichtungsmodul (50) so konfiguriert ist, dass es die Importdaten und die Scandaten ausrichtet, indem es gemeinsame Punkte auf der Grundlage der Importdaten und der Merkmalsinformationen der Scandaten identifiziert und die gemeinsamen Punkte einander zuordnet.

14. Dreidimensionales Scandatenverarbeitungssystem nach Anspruch 13, wobei das Extraktionsmodul (40) die Merkmalsinformationen der dreidimensionalen Form des Objekts aus den Importdaten erzeugt, und
die Merkmalsinformationen mindestens eine Information umfassen, die aus einer Gruppe ausgewählt ist, die aus den Oberflächenkrümmungsinformationen, den Oberflächenwelleninformationen und den Benutzereingabeinformationen besteht.

## Revendications

1. Procédé de traitement de données de numérisation tridimensionnelle comprenant :
une étape de réception de données d'importation (S100) dans laquelle un dispositif de traitement de données (20) importe des données d'importation comprenant des informations de forme d'au moins une partie d'un objet ;
une étape de réception de données de numérisation (S200) dans laquelle un module de réception de données de numérisation (10) génère, par numérisation de l'objet avec un scanner tridimensionnel (71), des données de numérisation qui comprennent des informations de forme et des informations de caractéristique de l'objet et qui sont au moins partiellement en commun avec les données d'importation ;
une étape d'extraction (S600) dans laquelle un module d'extraction (40) extrait, à partir des données d'importation, une région commune aux données de numérisation ;
une étape d'alignement (S700) dans laquelle un module d'alignement (50) effectue un alignement sur la base de la région commune aux données d'importation et aux données de numérisation ; et
une étape d'intégration (S900) dans laquelle un module d'intégration (70) génère des données intégrées en intégrant les données d'importation et les données de numérisation,
dans lequel, lors de l'étape d'extraction (S600), le module d'extraction (40) génère au moins une information sélectionnée parmi un groupe d'informations de courbure de surface et d'informations d'ondulation de surface formées en reliant des points adjacents des données d'importation, en tant qu'informations de caractéristique d'une forme tridimensionnelle de l'objet, et
dans lequel, lors de l'étape d'alignement (S700), les données d'importation et les données de numérisation sont alignées les unes avec les autres en identifiant des points communs sur la base des données d'importation et des informations de caractéristique des données de numérisation et en mappant les points communs les uns aux autres.

2. Procédé de traitement de données de numérisation tridimensionnelle selon la revendication 1, dans lequel l'étape d'extraction (S600) comprend la génération, par le module d'extraction (40), des informations de caractéristique de la forme tridimensionnelle de l'objet à partir des données d'importation, et
les informations de caractéristique comprennent au moins une information sélectionnée parmi un groupe des informations de courbure de surface, des informations d'ondulation de surface, et des informations d'entrée utilisateur.

3. Procédé de traitement de données de numérisation tridimensionnelle selon la revendication 1, comprenant en outre une étape d'affichage (S800) dans laquelle un module d'affichage (60) affiche, sur un dispositif d'affichage (51), la forme tridimensionnelle de l'objet définie par au moins une donnée sélectionnée parmi un groupe des données d'importation et des données de numérisation.

4. Procédé de traitement de données de numérisation tridimensionnelle selon la revendication 3, comprenant en outre une étape d'édition dans laquelle un outil d'édition (52) reçoit une commande d'édition pour éditer une partie des données d'importation et met à jour les données d'importation,
dans lequel l'étape d'intégration (S900) est effectuée après que l'étape d'édition a été effectuée.

5. Procédé de traitement de données de numérisation tridimensionnelle selon la revendication 3, dans lequel lors de l'étape d'affichage (S800), le module d'affichage (60) affiche sélectivement les formes tridimensionnelles de l'objet, qui sont définies par les données d'importation et les données de numérisation, sur le dispositif d'affichage (51) conformément à une commande reçue par le biais d'un dispositif d'entrée.

6. Procédé de traitement de données de numérisation tridimensionnelle selon la revendication 3, dans lequel lors de l'étape d'affichage (S800), le module d'affichage (60) voxélise chacune des données d'importation et des données de numérisation et affiche les données voxélisées sur le dispositif d'affichage (51).

7. Procédé de traitement de données de numérisation tridimensionnelle selon la revendication 3, dans lequel lors de l'étape d'affichage (S800), les formes tridimensionnelles de l'objet, qui sont définies par les données d'importation et les données de numérisation, sont affichées sur le dispositif d'affichage (51) dans des couleurs différentes.

8. Procédé de traitement de données de numérisation tridimensionnelle selon la revendication 3, dans lequel lors de l'étape d'affichage, le module d'affichage (60) affiche chaque région sur le dispositif d'affichage (51) dans une couleur différente en fonction de la fiabilité des données, et le module d'affichage (60) affiche une partie correspondant aux données d'importation sur le dispositif d'affichage (51) en fonction d'une fiabilité prédéterminée, et
la fiabilité des données d'importation est fixée à une valeur la plus élevée dans une plage de valeurs numériques prédéterminées.

9. Procédé de traitement de données de numérisation tridimensionnelle selon la revendication 1, dans lequel, lors de l'étape de réception de données d'importation (SI00), le dispositif de traitement de données (20) reçoit en outre, en tant que données d'importation, au moins un élément sélectionné parmi un groupe d'un vecteur normal indiquant des directions de points des données d'importation et de maillages formés en reliant les points.

10. Procédé de traitement de données de numérisation tridimensionnelle selon la revendication 1, comprenant en outre :
une étape de détermination de résolution (S400) dans laquelle un module de résolution (30) détermine si une distance entre des points adjacents des données d'importation dépasse une distance de référence, qui est une distance maximale autorisée entre des points adjacents des données de numérisation ; et
une étape de mise à jour des données d'importation (S500) dans laquelle, lorsqu'il est déterminé que la distance entre les points adjacents des données d'importation dépasse la distance de référence lors de l'étape de détermination de résolution (S400), le module de résolution (30) met à jour les données d'importation en générant de nouveaux points entre au moins certains points de manière que la distance entre les points adjacents des données d'importation soit égale ou inférieure à la distance de référence,
dans lequel l'étape d'intégration (S900) est effectuée après que l'étape de détermination de résolution (S400) et l'étape de mise à jour des données d'importation (S500) ont été effectuées.

11. Procédé de traitement de données de numérisation tridimensionnelle selon la revendication 1, comprenant en outre une étape de réception de points communs dans laquelle un module d'entrée reçoit, par l'intermédiaire d'un dispositif d'entrée, au moins un point commun à des endroits identiques entre eux dans les formes tridimensionnelles de l'objet définies par les données d'importation et les données de numérisation,
dans lequel, lors de l'étape d'alignement (S700), le module d'alignement (50) aligne les données d'importation et les données de numérisation les unes avec les autres par un procédé dans lequel le module d'entrée mappe le point commun des données d'importation et des données de numérisation qui est reçu lors de l'étape de réception de points communs .

12. Procédé de traitement de données de numérisation tridimensionnelle selon la revendication 1, dans lequel les données de numérisation générées par le module de réception de données de numérisation (10) lors de l'étape de réception de données de numérisation (S200) sont des données accumulées en temps réel par le scanner tridimensionnel (71),
l'étape d'alignement (S700) et l'étape d'intégration (S900) sont effectuées tandis que les données de numérisation accumulées en temps réel par l'étape de réception de données de numérisation (S200) sont mises à jour, et
lors de l'étape de réception des données d'importation (SI00), le dispositif de traitement de données (20) reçoit les données d'importation sans utiliser le scanner tridimensionnel (71).

13. Système de traitement de données de numérisation tridimensionnelle comprenant :
un dispositif de traitement de données (20) configuré pour importer des données d'importation définissant une forme tridimensionnelle comportant des informations de forme d'au moins une partie d'un objet ;
un module de réception de données de numérisation (10) configuré pour générer, par numérisation de l'objet avec un scanner tridimensionnel, des données de numérisation qui comprennent des informations de forme et des informations de caractéristique de l'objet et qui sont au moins partiellement en commun avec les données d'importation ;
un module d'extraction (40) configuré pour extraire une région commune aux données de numérisation à partir des données d'importation ;
un module d'alignement (50) configuré pour effectuer un alignement sur la base de la région commune aux données d'importation et aux données de numérisation ; et
un module d'intégration (70) configuré pour générer des données intégrées en intégrant les données d'importation et les données de numérisation,
dans lequel le module d'extraction (40) est configuré pour générer au moins une information sélectionnée parmi un groupe d'informations de courbure de surface et d'informations d'ondulation de surface formées en reliant des points adjacents des données d'importation, en tant qu'informations de caractéristique d'une forme tridimensionnelle de l'objet, et
dans lequel le module d'alignement (50) est configuré pour aligner les données d'importation et les données de numérisation en identifiant des points communs sur la base des données d'importation et des informations de caractéristique des données de numérisation et en mappant les points communs les uns aux autres.

14. Système de traitement de données de numérisation tridimensionnelle selon la revendication 13, dans lequel le module d'extraction (40) génère les informations de caractéristique de la forme tridimensionnelle de l'objet à partir des données d'importation, et
les informations de caractéristique comprennent au moins une information sélectionnée parmi un groupe des informations de courbure de surface, des informations d'ondulation de surface, et des informations d'entrée utilisateur.
